# EUROPÄISCHE PATENTSCHRIFT

(11) **EP 2 907 582 B1**
(45) Veröffentlichungstag und Bekanntmachung des Hinweises auf die Patenterteilung: **06.09.2017**
(21) Anmeldenummer: 14155410.5
(22) Anmeldetag: 17.02.2014
(51) Int. Cl.: B05B 7/08, B05B 7/10, B05B 7/04, A61M 11/08, B01F 5/04, B01F 5/20, A61B 17/00

(54) **Verfahren und Düse zum Mischen und Versprühen von medizinischen Fluiden**
Method and nozzle for mixing and spraying medical fluids
Procédé et buse permettant de mélanger et de pulvériser des fluides médicaux

(43) Veröffentlichungstag der Anmeldung: 19.08.2015
(73) Patentinhaber: Erbe Elektromedizin GmbH, 72072 Tübingen (DE)
(72) Erfinder: Fech, Andreas, 72072 Tübingen (DE); Fischer, Klaus, 72202 Nagold (DE); Enderle, Markus, Dr., 72070 Tübingen (DE)
(74) Vertreter: Bohnenberger, Johannes

(56) Entgegenhaltungen:
- WO-A1-90/01959
- WO-A1-98/13094
- WO-A1-99/17833
- GB-A- 2 033 251
- JP-A- H05 132 503
- JP-A- 2011 194 304
- US-A1- 2012 305 669

## Beschreibung

Die Erfindung betrifft ein Verfahren zum Mischen von wenigstens zwei Fluiden mittels einer außenmischenden Düse für medizinische Zwecke. Ferner betrifft die Erfindung eine außenmischende Düse zur Zufuhr von Substanzen, insbesondere biologischem Material sowie ein medizinisches Instrument und ein medizinisches Gerät mit einer derartigen Düse. Eine Düse gemäß dem Oberbegriff des Patentanspruchs 7 ist beispielsweise aus US 5,368,563 A bekannt.

Bei der bekannten Düse sind zwei Drallkammern vorgesehen, in die jeweils ein Austrittskanal mündet. Über die Drallkammern werden zwei unterschiedliche Fluide in Rotationsbewegung versetzt, bevor die Fluide die Düse über die Austrittskanäle verlassen. Wegen des zuvor auf die Fluide aufgebrachten Dralls ergibt sich ein rotierender Sprühstrahl. Dabei sind die beiden Austrittsöffnungen der bekannten Düse derart beabstandet voneinander angeordnet, dass sich die beiden rotierenden Fluidkegel überlappen, so dass außerhalb der Düse eine Vermischung der beiden Fluide erfolgt.

Aufgrund der offenbarten Merkmale der bekannten Düse ist eine gleichmäßige Mischung von Fluiden, die unterschiedliche Viskosität aufweisen und/oder mit unterschiedlichen Volumenströmen zugeführt werden, nicht mit der gewünschten Qualität erreichbar, beziehungsweise gar nicht möglich. Ein weiterer Nachteil der bekannten Düse besteht darin, dass die Fluide in den Drallkammern hohen Belas-Belastungen ausgesetzt sind, was bei der Zufuhr von biologischem Material, beispielsweise Zellen, unerwünscht ist.

Die Aufgabe der Erfindung besteht darin, ein Verfahren zum Mischen von wenigstens zwei Fluiden mittels einer außenmischenden Düse für medizinische Zwecke anzugeben, das eine gleichmäßige Vermischung von Fluiden ermöglicht. Ferner besteht die Aufgabe der Erfindung darin, eine außenmischende Düse anzugeben, die ein gleichmäßiges Vermischen von Fluiden erlaubt und insbesondere eine schonende Zumischung eines biologischen Materials ermöglicht. Schließlich besteht die Aufgabe der Erfindung darin, ein medizinisches Instrument und ein medizinisches Gerät mit einer derartigen außenmischenden Düse anzugeben.

Erfindungsgemäß wird diese Aufgabe im Hinblick auf das Verfahren durch den Gegenstand des Patentanspruchs 1, im Hinblick auf die außenmischende Düse durch den Gegenstand des Patentanspruchs 7, im Hinblick auf das medizinische Instrument durch den Gegenstand des Patentanspruchs 13 und im Hinblick auf das medizinische Gerät durch den Gegenstand des Patentanspruchs 14 gelöst.

Die Erfindung beruht auf dem Gedanken, ein Verfahren zum Mischen von wenigstens zwei Fluiden mittels einer außenmischenden Düse für medizinische Zwecke anzugeben, die wenigstens zwei Austrittskanäle, aus denen die Fluide aus der Düse austreten können, und wenigstens zwei Eintrittsöffnungen, durch die die Fluide in Kammern, insbesondere Misch- oder Drallkammern, eintreten können, aufweist. Damit die Fluide außerhalb der Düse gemischt werden, kann es zweckmäßig sein, wenn wenigstens eines der zu mischenden Fluide in einem kegelförmigen Strahl aus der Düse austritt.

Die wenigstens zwei Austrittskanäle und/oder die wenigstens zwei Eintrittsöffnungen können unterschiedliche oder gleiche Querschnitte aufweisen. Insbesondere können sich die Querschnitte der Eintrittsöffnungen voneinander unterscheiden oder identisch sein. Ebenso können sich die Querschnitte der Austrittskanäle voneinander unterscheiden oder identisch sein. Es ist auch möglich, dass sich der Querschnitt eines Austrittskanals von einem Querschnitt einer Eintrittsöffnung, die diesem Austrittskanal zugeordnet ist, unterscheidet oder diese Querschnitte identisch sind. Ferner können die Durchmesser der Kammern unterschiedlich groß sein. Bei dem erfindungsgemäßen Verfahren wird ein erstes Fluid über einen ersten Zufuhrkanal seitlich, insbesondere tangential, in eine erste Kammer geleitet,
die mit einem ersten Austrittskanal fluidverbunden ist. Ein zweites Fluid wird über einen zweiten Zufuhrkanal seitlich, insbesondere tangential, in eine zweite Kammer geleitet, die mit einem zweiten Austrittskanal fluidverbunden ist. Das erste Fluid strömt über den ersten Austrittskanal und das zweite Fluid über den zweiten Austrittskanal aus, so dass sich überlappende Fluidkegel bilden. Der Überlappungsgrad der Fluidkegel kann abhängig von der beabsichtigten Anwendung ausgebildet sein. So ist es möglich, dass der überlappende Bereich der beiden Fluidkegel sich aus gleichen Anteilen der beiden Fluidkegel bildet. Es ist jedoch auch möglich, dass der überlappende Bereich zum größten Teil nur von einem Fluidkegel gebildet wird. Die Fluidkegel werden von den Merkmalen der bei dem Verfahren eingesetzten Düse sowie der zugeführten Fluide bestimmt. Dabei können das erste Fluid und das zweite Fluid unterschiedliche Viskosität aufweisen und mit unterschiedlichen Volumenströmen, d.h. in einem bestimmten Volumenstromverhältnis, zugeführt werden. Ferner entspricht das Verhältnis der Querschnitte der Eintrittsöffnungen und/oder der Austrittskanäle dem Verhältnis der Volumenströme. Beispielsweise können das erste Fluid und das zweite Fluid mit im Wesentlichen gleichen Strömungsgeschwindigkeiten durch die Austrittskanäle und/oder die Eintrittsöffnungen fließen. Zusätzlich können in besonderen Anwendungsfällen die Kammern unterschiedliche Durchmesser aufweisen. Durch eine entsprechend ausgebildete Düse kann sichergestellt werden, dass es trotz unterschiedlicher Volumenströme des ersten Fluids und des zweiten Fluids zur Ausbildung von Fluidkegeln mit einem ausreichend großem Öffnungswinkel kommt. Dies kann annähernd gleich große Fluidkegelbereiche, die sich überlappen, ergeben.

Mit dem erfindungsgemäßen Verfahren wird erreicht, dass sich in den überlappenden Fluidkegeln eine gleichmäßige Vermischung der unterschiedlichen Fluide einstellt. Da die Strömungsgeschwindigkeiten der beiden Fluide trotz ihrer unterschiedlichen Viskosität oder Volumenströme aneinander angeglichen sind, wird eine gute und konstante Vermischung der Fluide erreicht. Dabei erfolgt die Vermischung außerhalb der Düse, so dass die Gefahr einer Düsenverstopfung vermieden wird. Innerhalb der Düse werden die Fluide vorzugsweise vollständig getrennt voneinander geführt. Konkret kann die Vernetzungsreaktion bzw. Vermischung der Fluide ausschließlich außerhalb der Düse erfolgen.

Vorzugsweise ist die für das erfindungsgemäße Verfahren eingesetzte Düse speziell an die Eigenschaften zweier ausgewählter Fluide angepasst. Für jede gewünschte Fluidkombination kann so eine entsprechend adaptierte Düse bereitgestellt werden. Dabei unterscheiden sich die Düsen beispielsweise durch die Querschnitte der Austrittskanäle und/oder der Eintrittsöffnungen in die Kammern, und/oder die Kammerdurchmesser. Durch diese geometrischen Variablen ist es möglich, die Strömungsgeschwindigkeiten für vorbestimmte Fluide gezielt einzustellen. Dabei kann die außenmischende Düse sowohl zwei Austrittskanäle mit unterschiedlichen Querschnitten, als auch zwei Eintrittsöffnungen mit unterschiedlichen Querschnitten und oder unterschiedlichen Kammerdurchmesser aufweisen. Es ist auch möglich, dass die Querschnitte der Austrittskanäle und/oder der Eintrittsöffnungen in die Kammern gleich sind. Die Angleichung der Strömungsgeschwindigkeiten kann dann beispielsweise über eine Steuerung erfolgen, die den Fluiddruck und/oder die Volumenströme der Fluide beeinflusst. Mit anderen Worten ist in einer bevorzugten Ausgestaltung des erfindungsgemäßen Verfahrens vorgesehen, dass die Zufuhr des ersten Fluids zur ersten Kammer und/oder die Zufuhr des zweiten Fluids zur zweiten Kammer gesteuert wird.

In diesem Zusammenhang wird darauf hingewiesen, dass der Durchmesser der Kammer sich auf die Dimension der Kammer in einer Ebene senkrecht zur Mittelachse der Kammer bezieht. Bei der Erfindung kann die Kammer insbesondere die Funktion übernehmen, einen Drall auf die über die Eintrittsöffnungen einströmenden Fluide auszuüben. Dazu weist die Kammer vorzugsweise einen runden, insbesondere kreisrunden, Querschnitt auf. Der Durchmesser des runden Kammerquerschnitts wird im Rahmen der vorliegenden Anmeldung als Kammerdurchmesser bezeichnet.

In einer weiteren Ausgestaltung des erfindungsgemäßen Verfahrens kann ein drittes Fluid durch einen dritten Zufuhrkanal in die zweite Kammer geleitet und mit dem zweiten Fluid vermischt werden. Das dritte Fluid kann also bereits vor dem Austritt aus der Austrittsöffnung mit dem zweiten Fluid vermischt werden. Bevorzugt ist vorgesehen, dass das zweite Fluid und das dritte Fluid keine oder eine nur geringe Vernetzungsreaktion miteinander eingehen. Hingegen kann das erste Fluid eine Vernetzungsfunktion aufweisen, so dass durch Mischung des ersten Fluids mit dem Gemisch aus dem zweiten und dritten Fluid außerhalb der Düse eine Vernetzung erfolgt. Die Vernetzung aller Fluide erfolgt vorzugsweise außerhalb der Düse, beispielsweise erst durch Zugabe des ersten Fluids zum Gemisch aus dem zweiten und dritten Fluid.

Um die Belastung, der die Fluide ausgesetzt sind, zu verringern, beispielsweise um Scherkräfte beim Eintritt des dritten Fluids in die zweite Kammer zu reduzieren, kann vorteilhaft vorgesehen sein, dass das dritte Fluid koaxial in die zweite Kammer einströmt. Insbesondere kann das dritte Fluid koaxial zur zweiten Kammer in die zweite Kammer einströmen, wobei die zweite Kammer koaxial mit dem zweiten Austrittskanal verbunden ist. Folglich kann das dritte Fluid koaxial zum zweiten Austrittskanal die zweite Kammer durchströmen. So wird weitgehend vermieden, dass das dritte Fluid in der zweiten Kammer einem Drall und/oder einem erhöhten Druck ausgesetzt wird, der erhöhte Belastungen, insbesondere Scherkräfte bzw. Druck, zur Folge hätte. Der Einfluss von Scherkräften und/oder Druck auf das dritte Fluid wird minimiert, so dass eine besonders schonende Zufuhr bzw. Zumischung des dritten Fluids erfolgt. Das dritte Fluid kann beispielsweise ein biologisches Material, insbesondere Zellen enthalten, die eine hohe Sensibilität hinsichtlich Belastungen aufweisen. Das biologische Material wird durch die koaxiale Zuführung des dritten Fluids geschont.

In einer alternativen Variante des erfindungsgemäßen Verfahrens kann ein drittes Fluid über einen dritten Austrittskanal in die überlappenden Fluidkegel des ersten Fluids und des zweiten Fluids geleitet werden. In dieser Variante ist der dritte Austrittskanal vorzugsweise zwischen dem ersten Austrittskanal und dem zweiten Austrittskanal positioniert, so dass das dritte Fluid in die Mischungs- bzw. Überlappungszone der Fluidkegel des ersten Fluids und des zweiten Fluids geleitet wird. Das dritte Fluid wird auf diese Weise außerhalb der Düse mit dem ersten Fluid und dem zweiten Fluid in Verbindung gebracht, so dass die Vermischung aller drei Fluide vollständig außerhalb der Düse erfolgt. Dies vermeidet eine Verstopfung einzelner Austrittskanäle oder anderer Komponenten der Düse. Zudem bewirkt das Zusammenführen der Fluide außerhalb der Düse eine besonders schonende Vermischung dieser Fluide.

Eine weitere alternative Ausführungsform des erfindungsgemäßen Verfahrens sieht vor, dass das dritte Fluid direkt, insbesondere seitlich, in den zweiten Austrittskanal eingeleitet wird. Dabei wird das dritte Fluid von der Strömung des zweiten Fluids im zweiten Austrittskanal angesaugt (Venturi-Prinzip) und gelangt so im Wesentlichen ohne Druckbelastung in den zweiten Austrittskanal, wobei zumindest teilweise eine Mischung des dritten Fluids mit dem zweiten Fluid im Austrittskanal stattfindet. Das dritte Fluid wird also dem zweiten Fluid unmittelbar vor dem Austritt aus der Düse zugemischt, wodurch das Risiko einer Düsenverstopfung reduziert ist. Gleichzeitig wird eine gewünschte Vermischung des dritten Fluids mit dem zweiten Fluid erreicht.

Die vorliegende Erfindung beruht ferner auf dem Gedanken, eine außenmischende Düse zur Zufuhr von Substanzen, insbesondere biologischem Material, mit einem ersten Austrittskanal und einem zweiten Austrittskanal anzugeben. Der erste Austrittskanal und der zweite Austrittskanal sind beabstandet zueinander angeordnet, so dass sich aus den ersten und zweiten Austrittskanälen austretende Fluidkegel zur Vermischung von Fluiden zumindest teilweise überlappen. Der erste Austrittskanal ist mit einer ersten Kammer fluidverbunden. Der zweite Austrittskanal ist mit einer zweiten Kammer fluidverbunden. Ferner mündet ein erster Zufuhrkanal seitlich, insbesondere tangential, in die erste Kammer. Ein zweiter Zufuhrkanal mündet seitlich, insbesondere tangential, in die zweite Kammer. Erfindungsgemäß ist wenigstens ein dritter Zufuhrkanal vorgesehen, der koaxial in die zweite Kammer oder direkt in den zweiten Austrittskanal oder einen dritten Austrittskanal mündet.

Die erfindungsgemäße Düse ermöglicht die Zufuhr und Vermischung von wenigstens drei Fluiden, wobei die Vermischung von wenigstens zwei Fluiden vollständig außerhalb der Düse erfolgt. Damit wird ein Verkleben oder Verstopfen der Düse effizient vermieden.

Der dritte Zufuhrkanal kann insbesondere seitlich in den zweiten Austrittskanal münden. Mit anderen Worten kann das im dritten Zufuhrkanal geführte dritte Fluid senkrecht zur Strömungsrichtung des zweiten Fluids, das im zweiten Austrittskanal geführt ist, zugemischt werden. Diese Art der Zumischung ist besonders effektiv und gleichzeitig schonend für Substanzen, insbesondere biologisches Material, das gegebenenfalls im dritten Fluid enthalten sein kann.

In einer bevorzugten Variante der erfindungsgemäßen Düse ist der dritte Austrittskanal zwischen dem ersten und dem zweiten Austrittskanal angeordnet, so dass ein aus dem dritten Austrittskanal austretendes Fluid, insbesondere das dritte Fluid, in die überlappenden Fluidkegel geleitet wird. In dieser Variante erfolgt die Vermischung der wenigstens drei Fluide vollständig außerhalb der Düse, was eine Düsenverstopfung sicher vermeidet. Durch die zentrale Anordnung des dritten Austrittskanals zwischen dem ersten und dem zweiten Austrittskanal wird die Belastung z.B. durch Scherkräfte auf das aus dem dritten Austrittskanal austretende dritte Fluid reduziert.

Bei der erfindungsgemäßen Düse kann auch vorgesehen sein, dass der dritte Zufuhrkanal koaxial zum zweiten Austrittskanal angeordnet ist. Das im dritten Zufuhrkanal geführte dritte Fluid gelangt somit koaxial bzw. zentral in die zweite Kammer und kann dort schonend mit einem zweiten Fluid gemischt werden, das über den zweiten Zufuhrkanal in die zweite Kammer einströmt. Durch die seitliche Einmündung des zweiten Zufuhrkanals entsteht in der zweiten Kammer ein Drall, der zu einer Verwirbelung führt, so dass das dritte Fluid, das über den koaxial angeordneten dritten Zufuhrkanal einströmt, gut mit dem zweiten Fluid vermischt wird. Gleichzeitig bewirkt die koaxiale Anordnung des dritten Zufuhrkanals eine Schonung des dritten Fluids, insbesondere hinsichtlich auftretender Belastungen, insbesondere Scherkräfte und/oder Druck.

Um eine Anpassung der Strömungsgeschwindigkeiten der aus den Austrittskanälen austretenden Fluide und somit eine Angleichung der Sprühkegel hinsichtlich der Geschwindigkeitskomponenten (axial, radial) für eine bestmögliche homogene Verteilung der Fluide sowie eine größtmögliche Überlappung der Sprühkegel zu erreichen, kann bevorzugt vorgesehen sein, dass die erste Kammer und die zweite Kammer jeweils mit dem ersten oder zweiten Zufuhrkanal über eine Eintrittsöffnung verbunden sind, wobei die Eintrittsöffnung der ersten und die Eintrittsöffnung der zweiten Kammer unterschiedliche Querschnittsflächen aufweisen. Die Querschnittsflächen der Eintrittsöffnungen der ersten Kammer bzw. der zweiten Kammer können sich in ihrer geometrischen Form und/oder ihrer Größe unterscheiden. Vorzugsweise sind die Querschnittsflächen unterschiedlich groß, wobei die Größendifferenz zwischen den Querschnittsflächen derart angepasst ist, dass sich die Strömungsgeschwindigkeiten der in die Kammer einströmenden Fluide angleichen.

Schließlich kann bei der erfindungsgemäßen Düse vorgesehen sein, dass wenigstens der erste Austrittskanal und der zweite Austrittskanal, insbesondere alle Austrittskanäle, parallel oder winklig zueinander ausgerichtete Längsachsen aufweisen. Eine parallele Ausrichtung der Längsachsen der Austrittskanäle erleichtert die Herstellung der erfindungsgemäßen Düse. Durch eine winklige Ausrichtung der Längsachsen der Austrittskanäle wird eine Vergrößerung des Überlappungsbereichs der Fluidkegel erreicht. Dies unterstützt die Vermischung der Fluide und verbessert so die Mischfunktiön der Düse.

Außerdem kann gemäß einem bevorzugten Ausführungsbeispiel der erfindungsgemäßen Düse vorgesehen sein, dass der zweite Austrittskanal eine Engstelle aufweist. Dabei kann der dritte Zufuhrkanal insbesondere im Bereich der Engstelle in den zweiten Austrittskanal münden. Vorzugsweise mündet der dritte Zufuhrkanal seitlich'im Bereich der Engstelle in den zweiten Austrittskanal. Die Engstelle kann im Wesentlichen eine Verjüngung bilden, so dass der Austrittskanal an sich nach Art einer Venturidüse ausgebildet ist. Der Austrittskanal weist somit unterschiedliche bzw. variierende Querschnittsdurchmesser auf, wobei der Querschnitt des Austrittskanals vorzugsweise kreisförmig ist. Es wird darauf hingewiesen, dass nicht nur der zweite Austrittskanal, sondern auch der erste Austrittskanal und/oder der dritte Austrittskanal nach Art einer Venturidüse ausgebildet sein können, also im Wesentlichen eine Engstelle aufweisen können. Die Engstelle bildet den kleinsten Querschnittsdurchmesser des jeweiligen Austrittskanals. Bei dem Vergleich der Querschnittsdimensionen der Eintrittsöffnungen und der Austrittskanäle wird hinsichtlich der Austrittskanäle, die nach Art einer Venturidüse ausgebildet sind, der kleinste Querschnittsdurchmesser im Bereich der Engstelle herangezogen. Der kleinste Querschnittsdurchmesser kann beispielsweise mit Hilfe eines Messzylinders ermittelt werden, der durch den Austrittskanal geführt wird. Der Durchmesser des maximal größten Messzylinders, der durch den Austrittskanal geführt werden kann, entspricht dem Querschnittsdurchmesser des Austrittskanals an der Engstelle bzw. am kleinsten Querschnittsdurchmesser.

Im Bereich der Engstelle ist die Strömungsgeschwindigkeit des durch den Austrittskanal fließenden Fluids maximal, so dass gemäß dem Venturi-Prinzip im Zufuhrkanal, der im Bereich der Engstelle in den Austrittskanal mündet, ein Unterdruck erzeugt wird. Auf diese Weise wird das über den Zufuhrkanal in den Austrittskanal seitlich eingeleitete Fluid mit Hilfe des im Austrittskanal strömenden Fluids angesaugt, wodurch Druckbelastungen auf das zuströmende Fluid vermieden werden.

Ein nebengeordneter Aspekt der Erfindung betrifft ein medizinisches Instrument mit einer zuvor beschriebenen außenmischenden Düse, wobei das Instrument an ein medizinisches Gerät mit einer Steuerung oder Regelung zur Einstellung der Fluidzufuhr anschließbar ist.

Ferner wird im Rahmen der Erfindung ein medizinisches Gerät, das mit einer zuvor beschriebenen außenmischenden Düse und/oder einem solchen Instrument verbunden ist, beschrieben, wobei das medizinische Gerät eine Steuerung und/oder Regelung aufweist. Die Steuerung und/oder Regelung ist zur Einstellung der Fluidzufuhr ausgelegt, so dass bei unterschiedlichen Volumenströmen und/oder unterschiedlicher Viskosität der Fluide diese mit im Wesentlichen gleichen Strömungsgeschwindigkeiten durch die Austrittskanäle und/oder die Eintrittsöffnungen fließen.

Ferner kann ein medizinisches Gerät, das mit einer außenmischenden Düse und/oder einem zuvor beschriebenen Instrument verbunden ist, eine Steuerung oder Regelung aufweisen, die zur Einstellung der Fluidzufuhr derart ausgelegt ist, dass die unterschiedlichen Fluide unabhängig voneinander, in einer beliebigen Reihenfolge, beispielsweise in Intervallen zugeführt werden können. Es ist ferner möglich, dass ein medizinisches Gerät eine Steuerung oder Regelung aufweist, die derart zur Einstellung der Fluidzufuhr angepasst ist, dass sowohl die Strömungsgeschwindigkeiten der Fluide mit unterschiedlichen Volumenströmen und/oder unterschiedlicher Viskosität aneinander angeglichen, als auch die unterschiedlichen Fluide unabhängig voneinander, in einer beliebigen Reihenfolge zugeführt werden können. Dadurch kann erreicht werden, dass beispielsweise das erste Fluid aus dem ersten Austrittskanal aus der Düse austritt, auf ein Zielobjekt aufgesprüht wird, in einem zeitlichen Abstand dazu das zweite Fluid aus dem zweiten Austrittskanal aus der Düse austritt und die Vermischung beziehungsweise Vernetzung der beiden Fluide erst auf dem Zielobjekt beispielsweise einem Gewebe, insbesondere einem biologischen Gewebe, erfolgt.

Die Erfindung wird im Folgenden anhand von Ausführungsbeispielen unter Bezugnahme auf die beigefügten, schematischen Zeichnungen näher erläutert. Darin zeigen:
- Fig. 1:: eine Querschnittsansicht durch eine erfindungsgemäße außenmischende Düse gemäß einem bevorzugten Ausführungsbeispiel, wobei der dritte Zufuhrkanal koaxial in die zweite Kammer mündet;
- Fig. 2:: eine Querschnittsansicht einer erfindungsgemäßen außenmischenden Düse gemäß einem weiteren bevorzugten Ausführungsbeispiel, wobei der dritte Zufuhrkanal in einem dritten Austrittskanal mündet;
- Fig. 3:: eine Querschnittsansicht einer erfindungsgemäßen außenmischenden Düse nach einem weiteren bevorzugten Ausführungsbeispiel, wobei der dritte Zufuhrkanal seitlich in den zweiten Austrittskanal mündet;
- Fig.3a:: in einer vergrößerten Darstellung eine bevorzugte Ausgestaltung eines Austrittskanals einer erfindungsgemäßen außenmischenden Düse nach Fig.3;
- Fig. 4:: eine perspektivische Ansicht eines vorteilhaften Kanalsystems einer erfindungsgemäßen außenmischenden Düse in einteiliger Ausführung; und
- Fig. 5:: ein Zeitdiagramm mit einer beispielhaften Pulsfolge zur Ansteuerung des medizinischen Geräts im Intervallbetrieb

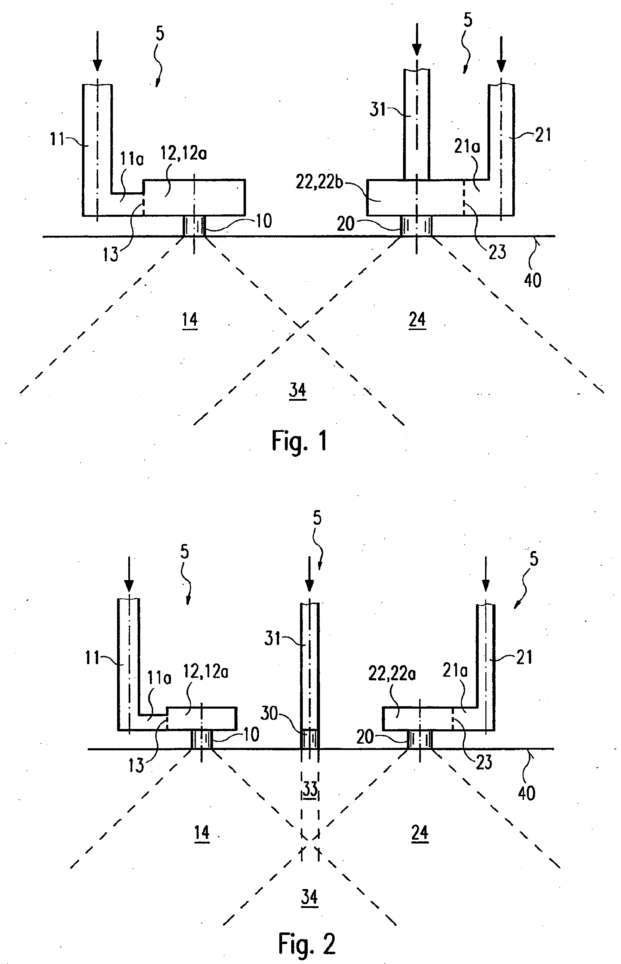

Fig. 1 zeigt im Querschnitt eine außenmischende Düse mit zwei verschiedenen Kanalsystemen 5. Dabei weist die Düse zwei Austrittskanäle 10, 20 auf. Die Austrittskanäle 10, 20 enden in einer Endfläche 40, die die Düse begrenzt. Die beiden Austrittskanäle 10, 20 bilden einen ersten Austrittskanal 10 und einen zweiten Austrittskanal 20. Der erste Austrittskanal 10 und der zweite Austrittskanal 20 sind beabstandet voneinander angeordnet. Bei dem dargestellten Ausführungsbeispiel ist deutlich sichtbar, dass die Austrittskanäle 10, 20 unterschiedliche Querschnitte, insbesondere Querschnittsdurchmesser, aufweisen. Vorzugsweise sind die Austrittskanäle 10, 20 kreiszylinderförmig ausgebildet, wobei der zweite Austrittskanal 20 einen größeren Querschnittsdurchmesser als der erste Austrittskanal 10 aufweist.

Der erste Austrittskanal 10 verbindet die Endfläche 40 mit einer Kammer 12, die insbesondere als Drallkammer 12a ausgebildet ist. Die Drallkammer 12a bewirkt eine Verwirbelung des zugeführten ersten Fluids, so dass sich beim Austritt des Fluids über den ersten Austrittskanal 10 ein kegelförmiger Fluidstrahl einstellt. Der Fluidkegel 14 des ersten Fluids ist in den Figuren durch gestrichelte Linien dargestellt.

Um das erste Fluid in der Drallkammer 12a in eine Rotationsbewegung zu versetzen, mündet ein erster Zufuhrkanal 11 seitlich in die Drallkammer 12a. Insbesondere kann der erste Zufuhrkanal 11 tangential in die erste Drallkammer 12a münden. Mit anderen Worten kann der erste Zufuhrkanal 11 eine Innenwand aufweisen, die nahtlos bzw. kontinuierlich oder absatzfrei in eine Innenwand der ersten Drallkammer 12a übergeht.

Der erste Zufuhrkanal 11 verläuft im Wesentlichen senkrecht zur Endfläche 40 durch die Düse und weist einen abgewinkelten Endabschnitt 11a auf, der im Wesentlichen parallel zur Endfläche 40 durch die Düse verläuft und in die erste Kammer 12, insbesondere die erste Drallkammer 12a, mündet. Im Mündungsbereich zwischen dem ersten Zufuhrkanal 11, insbesondere dessen Endabschnitt 11a, und der ersten Drallkammer 12a, weist der erste Zufuhrkanal 11 eine erste Eintrittsöffnung 13 auf. Die erste Eintrittsöffnung 13 weist eine Höhe auf, die kleiner als die Höhe der Drallkammer 12a ist. Der Querschnitt der ersten Eintrittsöffnung 13 kann in Abhängigkeit der Viskosität oder des Volumenstroms des zugeführten ersten Fluids gewählt werden. Beispielsweise kann die erste Eintrittsöffnung 13 eine Höhe aufweisen, die der Höhe der Drallkammer 12a entspricht.

Der zweite Austrittskanal 20 verbindet die Endfläche 40 mit einer zweiten Kammer 22, die bei dem Ausführungsbeispiel gemäß Fig. 1 als Mischkammer 22b ausgebildet ist. Ähnlich wie bei der ersten Kammer 12 mündet in die zweite Kammer 22 ein zweiter Zufuhrkanal 21. Der zweite Zufuhrkanal 21 verläuft im Wesentlichen parallel zum ersten Zufuhrkanal 11, also senkrecht zur Endfläche 40, und weist einen abgewinkelten Endabschnitt 21a auf, der in die Mischkammer 22b mündet. Der abgewinkelte Endabschnitt 21a des zweiten Zufuhrkanals 21 mündet insbesondere über eine zweite Eintrittsöffnung 23 in die zweite Kammer 22 bzw. die Mischkammer 22b. In den hier dargestellten Ausführungsbeispielen gemäß Figuren 1 und 2 weist die zweite Eintrittsöffnung 23 eine Höhe auf, die der Höhe der Mischkammer 22b bzw. allgemein der zweiten Kammer 22 entspricht. Eine andere Dimensionierung, insbesondere hinsichtlich der Querschnittsfläche, der zweiten Eintrittsöffnung 23 ist möglich und wird vom Fachmann in Abhängigkeit des Volumenstroms und der Viskosität des zugeführten zweiten Fluids gewählt.

Bei dem Ausführungsbeispiel gemäß Fig. 1 ist erkennbar, dass die zweite Eintrittsöffnung 23 einen größeren Querschnitt als die erste Eintrittsöffnung 13 aufweist. Dabei wird davon ausgegangen, dass sich nur die Höhen der beiden Eintrittsöffnungen unterscheiden. Die anderen die Querschnittsfläche bestimmenden Parameter der beiden Eintrittsöffnungen sind identisch. Grundsätzlich kann das Verhältnis zwischen den Eintrittsöffnungen 13, 23 unterschiedlich oder gleich gewählt werden, um die Strömungsgeschwindigkeit der Fluide beim Austritt aus den Austrittskanälen 10, 20 und/oder beim Durchgang durch die Eintrittsöffnungen 13, 23 einzustellen, insbesondere einander anzugleichen. Dabei kann nicht nur die Höhe der Eintrittsöffnungen 13, 23, sondern auch die Breite der Eintrittsöffnungen 13, 23, also die jeweilige Querschnittsfläche variiert werden. Im Allgemeinen können sich die Eintrittsöffnungen 13, 23 sowohl in ihrer geometrischen Form, als auch in ihren Dimensionen voneinander unterscheiden.

Die Düse weist ferner einen dritten Zufuhrkanal 31 auf, der sich im Wesentlichen parallel zum ersten und zweiten Zufuhrkanal 11, 21, also senkrecht zur Endfläche 40, erstreckt. Der dritte Zufuhrkanal 31 dient zur Zuführung eines dritten Fluids. Der dritte Zufuhrkanal 31 mündet bei dem Ausführungsbeispiel gemäß Fig. 1 direkt in die zweite Kammer 22 bzw. die Mischkammer 22b. Die zweite Kammer 22 bildet eine Mischkammer, da sowohl das zweite Fluid, als auch das dritte Fluid in die zweite Kammer 22 geleitet und darin vermischt werden. Der dritte Zufuhrkanal 31 mündet vorzugsweise koaxial zur zweiten Kammer 22 in die zweite Kammer 22. Insbesondere sind bei dem Ausführungsbeispiel gemäß Fig. 1 der dritte Zufuhrkanal 31, die zweite Kammer 22 bzw. Mischkammer 22b und der zweite Austrittskanal 20 koaxial zueinander angeordnet. Dadurch wird erreicht, dass das dritte Fluid im Wesentlichen umlenkungsfrei durch die Düse geführt wird und über den zweiten Austrittskanal 20 die Düse verlässt. Gleichzeitig findet in der Mischkammer 22b eine Vermischung des dritten Fluids mit dem zweiten Fluid statt, da das zweite Fluid in der Mischkammer 22 in einen Drall versetzt wird. Dies wird dadurch erreicht, dass der zweite Zufuhrkanal 21 seitlich in die Mischkammer 22b mündet. Insbesondere mündet der zweite Zufuhrkanal 21 tangential in die Mischkammer 22b. Analog zum ersten Zufuhrkanal 11 weist auch der zweite Zufuhrkanal 21 eine Innenfläche auf, die bündig, insbesondere absatzfrei, in eine Innenfläche der Mischkammer 22b übergeht.

Das in der Mischkammer 22b erzeugte Fluidgemisch aus dem zweiten Fluid und dem dritten Fluid tritt aus dem zweiten Austrittskanal 20 als zweiter Fluidkegel 24 aus.

Im Allgemeinen haben die erste Kammer 12 und die zweite Kammer 22 die Funktion, das zu versprühende Fluid in einen Drall zu versetzen, woraus sich beim Austritt der Fluide aus den Austrittskanälen 10, 20 ein kegelförmiger Sprühstrahl einstellt. Auf diese Weise ergeben sich zwei Fluidkegel 14, 24, die sich unmittelbar nach der Endfläche 40 ausbilden. Vorzugsweise sind die Austrittskanäle 10, 20 derart voneinander beabstandet angeordnet, dass sich die Fluidkegel 14, 24 überlappen und einen Überlappungsbereich 34 bilden, wobei der Überlappungsbereich 34 von der Endfläche 40 beabstandet angeordnet ist. Im Überlappungsbereich 34 erfolgt die Vermischung der Fluide aus den Fluidkegeln 14, 24. Durch den im Abstand zur Endfläche 40 der Düse angeordneten Überlappungsbereich 34 ist sichergestellt, dass Fluide, die in diesem Überlappungsbereich 34 gemischt werden, die Düse, insbesondere die Austrittskanäle 10 und 20 sowie die Drall- bzw. Mischkammern 12, 22, nicht verschließen.

Um die Vermischung der Fluide außerhalb der Düse zu verbessern, kann vorgesehen sein, dass die Austrittskanäle 10, 20, insbesondere der erste Austrittskanal 10 und der zweite Austrittskanal 20, winklig zueinander angeordnet sind. Die Längsachsen des ersten Austrittskanals 10 und des zweiten Austrittskanals 20 können also zueinander konvergieren, wobei der Schnittpunkt der Längsachsen außerhalb der Düse angeordnet ist. Daraus ergibt sich ein vergrößerter Überlappungsbereich 34. Bei den dargestellten Ausführungsbeispielen sind die Austrittskanäle 10, 20 parallel zueinander ausgerichtet, was Vorteile in der Herstellung der Düse hat.

Fig. 2 zeigt ein weiteres Ausführungsbeispiel der erfindungsgemäßen Düse mit drei unterschiedlichen Kanalsystemen 5 auf, wobei die Fluidführung für das erste Fluid (links in Figur 2 dargestellt) im Wesentlichen identisch zu der Düse gemäß Fig. 1 ausgebildet ist. Mit anderen Worten weist die Düse gemäß Fig. 2 einen ersten Austrittskanal 10 auf, der koaxial zu einer ersten Kammer 12, insbesondere einer Drallkammer 12a ausgerichtet ist. Der erste Austrittskanal 10 verbindet die erste Kammer 12 mit einer Endfläche 40 der Düse. In die erste Kammer 12 bzw. die erste Drallkammer 12a mündet seitlich ein erster Zufuhrkanal 11, der einen Endabschnitt 11a aufweist. Der Endabschnitt 11a umfasst eine erste Eintrittsöffnung 13 im Übergangsbereich zu ersten Drallkammer 12a. Wie beim Ausführungsbeispiel gemäß Figur 1 wird das erste Fluid in der ersten Drallkammer 12a in eine Rotationsbewegung versetzt, um beim Austritt des ersten Fluids aus dem ersten Austrittskanal 10 einen Fluidkegel 14 zu erzeugen.

Das zweite Fluid wird ebenfalls über eine Fluidführung an die Endfläche 40 geführt, die im Wesentlichen der Fluidführung gemäß Fig. 1 entspricht. So ist bei der Düse gemäß Fig. 2 ein zweiter Austrittskanal 20 vorgesehen, der koaxial zu einer zweiten Kammer 22 angeordnet ist und die zweite Kammer 22 mit der Endfläche 40 fluidverbindet. In die zweite Kammer 22 mündet ein zweiter Zufuhrkanal 21 über einen abgewinkelten Endabschnitt 21a und eine zweite Eintrittsöffnung 23. Der zweite Zufuhrkanal 21 mündet seitlich, insbesondere tangential, in die zweite Kammer 22. Im Unterschied zu dem Ausführungsbeispiel gemäß Fig. 1 ist die zweite Kammer 22 bei dem Ausführungsbeispiel gemäß Fig. 2 als zweite Drallkammer 22a ausgebildet. In der zweiten Drallkammer 22a erfolgt keine Fluidvermischung. Die -Kammern 12, 22 können unterschiedliche oder gleiche Durchmesser aufweisen. Das zweite Fluid wird in der zweiten Drallkammer 22a lediglich in eine Rotationsbewegung versetzt, um beim Austritt des zweiten Fluids aus dem zweiten Austrittskanal 20 einen Fluidkegel 24 zu erzeugen.

Bei dem Ausführungsbeispiel gemäß Fig. 2 ist ebenfalls erkennbar, dass der erste Austrittskanal 10 und der zweite Austrittskanal 20 unterschiedliche Querschnitte aufweisen. Jedenfalls weisen die erste Eintrittsöffnung 13 und die zweite Eintrittsöffnung 23 unterschiedliche Querschnitte auf. Dies ist, wie zuvor erläutert, variabel, um den Volumenströmen bzw. der Viskosität der unterschiedlichen Fluide insbesondere des ersten Fluids und des zweiten Fluids, gerecht zu werden. Zum Beispiel können durch die Einstellung eines entsprechenden Verhältnisses zwischen den Querschnitten der Austrittskanäle 10, 20 und den Querschnitten der Eintrittsöffnungen 13, 23 optimierte Strömungsgeschwindigkeiten, in einem besonderen Anwendungsfall gleiche Strömungsgeschwindigkeiten, beim Eintritt des ersten Fluids und des zweiten Fluids in die Drallkammer 12a, 22a und/oder beim Austritt des ersten Fluids und des zweiten Fluids aus den Austrittskanälen 10, 20 eingestellt werden. Eine optimierte Strömungsgeschwindigkeit des ersten Fluids und des zweiten Fluids gewährleistet einen optimalen Überlappungsbereich bei einer minimalen (geringstmöglichen) mechanischen Beanspruchung der zu applizierenden Materialien.

Bei dem Ausführungsbeispiel gemäß Fig. 2 ist ebenfalls ein dritter Zufuhrkanal 31 vorgesehen, der sich parallel zu dem ersten Zufuhrkanal 11 und dem zweiten Zufuhrkanal 21 erstreckt. Der dritte Zufuhrkanal 31 mündet direkt in einen dritten Austrittskanal 30, der zwischen dem ersten Austrittskanal 10 und dem zweiten Austrittskanal 20 angeordnet ist. Der dritte Zufuhrkanal 31 und der dritte Austrittskanal 30 sind koaxial zueinander ausgerichtet, so dass das dritte Fluid umlenkungsfrei aus der Düse austritt. Vorzugsweise tritt das dritte Fluid aus dem dritten Austrittskanal 30 als z.B. turbulenzarmer, Strahl 33 aus, der unmittelbar in den Überlappungsbereich 34 einströmt. So erfolgt die Vermischung aller drei Fluide erst im Überlappungsbereich 34, also außerhalb der Düse. Bei dem Strahl 33, der aus dem Austrittskanal 30 austritt, kann es sich um einen kohärenten, kontinuierlichen Flüssigkeitsaustritt oder einen unterbrochenen, in Tropfenform gebildeten Flüssigkeitsaustritt handeln.

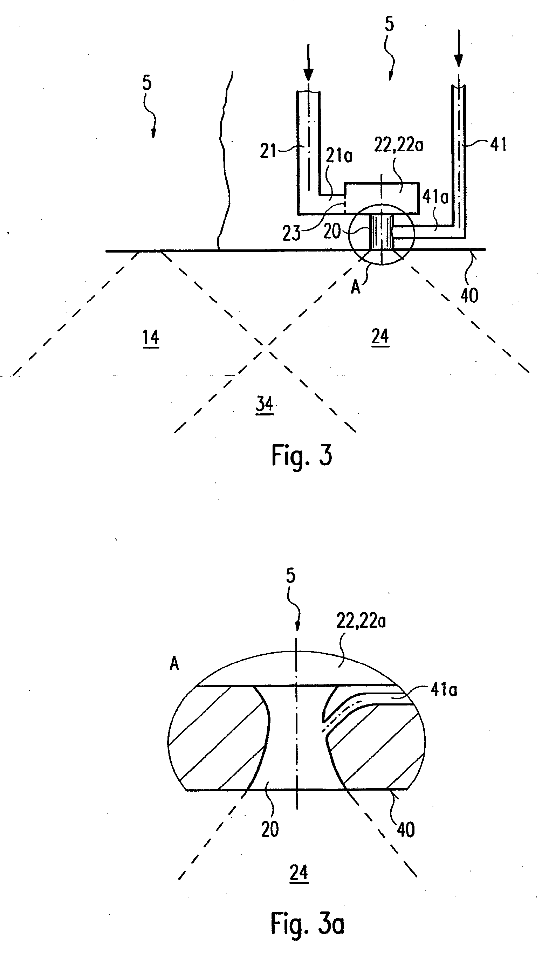

Ein weiteres Ausführungsbeispiel einer Düse ist in Fig. 3 gezeigt. Dabei ist aus Gründen der Übersichtlichkeit auf die Darstellung der Fluidführung des Kanalsystems 5 für das erste Fluid verzichtet worden. Lediglich der Fluidkegel 14 des ersten Fluids ist in Fig. 3 durch gestrichelte Linien gezeigt. Im Wesentlichen entspricht der nicht dargestellte Teil der Düse gemäß Fig. 3 dem entsprechenden Teil in den Figuren 1 und 2. Mit anderen Worten weist die Düse einen ersten Austrittskanal 10 auf, der die erste Kammer 12, insbesondere die erste Drallkammer 12a, mit der Endfläche 40 verbindet. In die Drallkammer 12a mündet seitlich der erste Zufuhrkanal 11 über die erste Eintrittsöffnung 13.

Die Düse gemäß Fig. 3 weist ferner ein weiteres Kanalsystem 5 mit einem zweiten Austrittskanal 20 auf, der an der Endfläche 40 endet. Der zweite Austrittskanal 20 entspringt einer zweiten Kammer 22, die als zweite Drallkammer 22a ausgestaltet ist. Die zweite Drallkammer 22a ist koaxial zum zweiten Austrittskanal 20 angeordnet. Seitlich mündet in die zweite Drallkammer 22a ein zweiter Zufuhrkanal 21 über einen zweiten Endabschnitt 21a, der im Bereich einer zweiten Eintrittsöffnung 23 in die zweite Drallkammer 22a übergeht. Vorzugsweise mündet der zweite Zufuhrkanal 21 tangential in die zweite Drallkammer 22a. Abgesehen vom abgewinkelten Endabschnitt 21a verläuft der zweite Zufuhrkanal 21 senkrecht zu der Endfläche 40.

Ebenfalls senkrecht zur Endfläche 40 erstreckt sich ein dritter Zufuhrkanal 41, der über einen abgewinkelten Endabschnitt 41a seitlich mit dem zweiten Austrittskanal 20 verbunden ist. Der Endabschnitt 41a des dritten Zufuhrkanals 41 erstreckt sich vorzugsweise parallel zur Endfläche 40 bzw. senkrecht zum zweiten Austrittskanal 20.

Bei dem Ausführungsbeispiel gemäß Fig. 3 ist erkennbar, dass die zweite Eintrittsöffnung 23 eine Höhe aufweist, die kleiner ist, als die Höhe der zweiten Drallkammer 22a. Im Allgemeinen kann der Querschnitt der zweiten Eintrittsöffnung 23 an die Viskosität oder den Volumenstrom des zweiten Fluids angepasst werden, um beim Austritt des zweiten Fluids aus der Düse eine Strömungsgeschwindigkeit des zweiten Fluids bzw. des Fluidgemischs aus dem zweiten und dritten Fluid einzustellen, die an die Strömungsgeschwindigkeit des aus dem ersten Austrittskanal 10 (in Fig 3 nicht dargestellt) ausströmenden ersten Fluids angepasst ist. Durch die mittels der Drallkammern 12a, 22a erzeugten Fluidkegel 14, 24 ergibt sich ein Überlappungsbereich 34, in dem die Fluide miteinander vermischt werden. Dabei erfolgt bei dem Ausführungsbeispiel gemäß Fig. 3 eine erste Vermischung des zweiten Fluids mit dem dritten Fluid bereits im zweiten Austrittskanal 20. Auch hier gilt wie bei den voran beschriebenen Ausführungsbeispielen, dass der Überlappungsbereich 34 von der Düse bzw. deren Endfläche 40 beabstandet angeordnet ist.

Eine weitere Ausführungsform der Düse ist in Fig. 3a dargestellt. In dem vergrößert dargestellten Ausschnitt ist die Ausgestaltung des Austrittkanals 20a sowie des Endabschnitts 41a des dritten Zufuhrkanals 41 dargestellt. In diesem Ausführungsbeispiel ist der Austrittskanal 20a im Unterschied zu den oben beschriebenen Ausführungsbeispielen nicht zylinderförmig mit gleichbleibendem Durchmesser ausgeführt, sondern nach Art einer Venturidüse ausgebildet. Das bedeutet, dass der Querschnitt des Austrittskanals 20a zwischen dessen proximalem und dessen distalem Ende einen variierenden Durchmesser aufweist. Konkret ist vorgesehen, dass der Austrittskanal 20a zwischen der Kammer 22 und der Endfläche 40 eine Engstelle 15 aufweist. Es ist weiterhin vorteilhaft vorgesehen, dass der Endabschnitt 41a in einem Winkel kleiner 90° zu der Längsachse des Austrittskanals 20a angeordnet ist. Dabei ist die Mündung des Endabschnitts 41a vorzugsweise an der engsten Stelle, d.h. im Bereich der Engstelle 15, des Austrittskanals 20a angeordnet. Diese Anordnung begünstigt die Erzeugung eines Unterdrucks in dem Endabschnitt 41a und somit eine Saugwirkung auf das dritte Fluid. Zudem begünstigt die Anordnung des Endabschnitts 41a in einem Winkel kleiner 90° einen schonenden Übergang des dritten Fluids in den zweiten Austrittskanal 20a. Zu einer verbesserten Verteilung und der Vermischung des zweiten und des dritten Fluids in dem zweiten Austrittskanal 20a können der dritte Zufuhrkanal 41 und der Endabschnitt 41a in mehrfacher Ausführung z.B. dreifach vorhanden sein (in Fig. 3a nicht gezeigt). Dabei können zumindest die Endabschnitte 41a symmetrisch um den Austrittskanal 20 angeordnet sein.

Eine erfindungsgemäße Düse kann jede mögliche Kombination der vorbeschriebenen Kanalsysteme zur Zuführung von Fluiden aufweisen. So sind Düsen mit wenigstens zwei Kanalsystemen 5 oder beispielsweise mit vier oder mehreren Kanalsystemen 5 möglich. Unabhängig von der Art und/oder Anzahl der verwendeten Kanalsysteme werden wenigstens zwei Fluide bei einer erfindungsgemäßen Düse in einem Überlappungsbereich 34, der außerhalb der Düse angeordnet ist, gemischt.

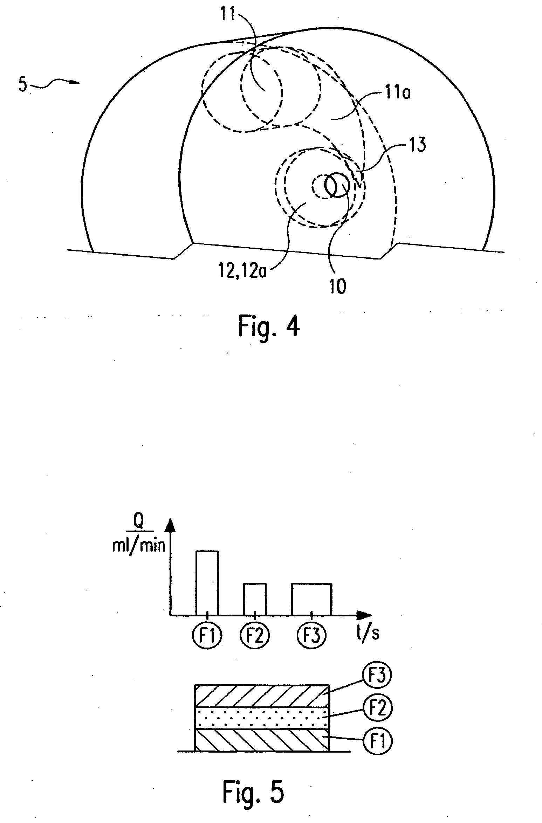

In einem weiteren Ausführungsbeispiel kann die Düse aus einem oder mehreren Kanalsystemen 5 gebildet sein, die jeweils einteilig ausgeführt sind. Es ist auch möglich, dass die gesamte Düse einteilig ausgebildet ist. Fig. 4 zeigt eine einteilige Ausführungsform eines Kanalsystems 5, wobei aus Gründen der Übersichtlichkeit auf die Darstellung der übrigen Kanalsysteme 5 verzichtet wurde. Dabei sind die Austrittskanäle 10, 20, 30, die Kammern 12, 22, die Eintrittsöffnungen 13, 23 sowie die Zufuhrkanäle 11, 21, 31 (in Fig. 4 gestrichelt dargestellt) integral in einem Bauteil ausgeführt. Vorzugsweise fließt bei dieser Ausführungsform der Düse das Fluid durch kantenfreie Umlenkungen bis in die Mischkammer. Dies bedeutet, dass die radial nach außen versetzten Zufuhrkanäle 11, 21, 31, rohrförmig gebildet sind. Im Unterschied zu den oben beschriebenen Ausführungsbeispielen, bei denen die Zufuhrkanäle 11, 21, insbesondere deren Umlenkbereiche, durch aneinandergefügte Flächenabschnitte gebildet sind, sind bei der einteilig ausgeführten Düse diese Umlenkbereiche durch zylinderförmige, kantenfreie Leitungsabschnitte gebildet. Dies ermöglicht auch bei Änderung der Fließrichtung um bis zu 90 Grad eine graduelle Umlenkung des zuzuführenden Fluids. Dadurch kann das zuzuführende Fluid schonend den Kammern 12, 22, zugeführt werden. Die Ausgestaltung der Zufuhrkanäle 11, 21 ermöglicht eine kontinuierliche, graduelle Umlenkung der Strömungsrichtung der durch diese Zufuhrkanäle 11, 13 strömenden Fluide vom proximalen Ende der Düse bis zum Eintritt in die Kammern 12, 22. Obwohl sich die Strömungsrichtung ausgehend vom distalen Ende der Zufuhrkanäle 40, 50, 51 bis in die Mischkammer um bis zu 90 Grad ändert, sind die Zufuhrkanäle 11, 12 vorzugsweise stufenlos bzw. mit kontinuierlich stufenlosen Krümmungen ausgebildet. Insbesondere weisen die Zufuhrkanäle 11, 12 auf dieser gesamten Strecke keine Stellen auf, die eine abrupte Umlenkung der Strömungsrichtung erzeugen.

Die im Wesentlich stufenlos ausgebildeten bzw. mit kontinuierlichen Krümmungen ausgestatteten Zufuhrkanäle 11, 12 eignen sich insbesondere zur Zuführung von biologischem Material, insbesondere Zellen. Bei dem Ausführungsbeispiel gemäß Fig. 4 mündet der erste Zufuhrkanal 11 über einen Endabschnitt 11a in die erste Kammer 12 bzw. erste Drallkammer 12a. Der Endabschnitt 11a des ersten Zufuhrkanals 11 weist kontinuierlich gekrümmte Seitenflächen auf, so dass durch den ersten Zufuhrkanal 11 strömendes Fluid schonend in einer kontinuierlichen Krümmung zur ersten Eintrittsöffnung 13 geführt wird. Dabei gehen die Seitenwände des Endabschnitts 11a des ersten Zufuhrkanals 11 im Wesentlichen stufenlos in die Seitenwände der ersten Kammer 12 über, so dass das in die erste Kammer 12 einströmende Fluid unmittelbar in eine Drallbewegung versetzt wird. Zwar eignet sich eine derartige Gestaltung des ersten Zufuhrkanals 11 mit einem gekrümmten Endabschnitt 11a und einem kontinuierlichen Übergang die Kammer 12 insbesondere zur Zufuhr von biologischen Zellen bzw. biologischen Zellen enthaltenden Fluiden. Das Kanalsystem 5 gemäß Fig. 4 ist jedoch auch für die Zufuhr anderer Materialien bzw. Fluide geeignet.

Die zuvor beschriebenen Varianten der erfindungsgemäßen Düse können jeweils mit einer Steuerung oder Regelung gekoppelt sein, um die Strömungsgeschwindigkeiten der Fluide beim Austritt aus der Düse einzustellen, insbesondere in Abhängigkeit der einzelnen Volumenströme und/oder der einzelnen Viskosität der Fluide. Dabei zielt die Steuerung bzw. Regelung darauf ab, eine einheitliche Strömungsgeschwindigkeit für alle Fluide einzustellen. Ferner kann die Steuerung oder Regelung ein Versprühen der Fluide bewirken, wobei die unterschiedlichen Fluide unabhängig voneinander und/oder in einer beliebigen Reihenfolge aus der Düse austreten. Mit anderen Worten kann die Düse derart angesteuert sein, dass aus den Austrittskanälen 10, 20, 30 sequentiell oder gleichzeitig Fluide austreten.

Fig. 5 zeigt beispielshaft eine mögliche Abfolge von Fluidzufuhrsequenzen zur Erzeugung eines mehrschichtigen Aufbaus unterschiedlicher Fluidauftragsschichten. So kann beispielsweise vorgesehen sein, dass ein erstes Fluid F1 zunächst mit einem hohen Volumenstrom auf ein Zielobjekt aufgetragen wird. In einem zeitlichen Abstand zum Auftragen des ersten Fluids F1 kann ein zweites Fluid F2 mit einem relativ kleineren Volumenstrom über die erfindungsgemäße Düse versprüht werden. Das erste Fluid F1 und das zweite Fluid F2 können jeweils über eine Sprühdauer, d.h. ein Zeitintervall von Start des Sprühstrahls bis Stopp des Sprühstrahls, versprüht werden, die identisch oder zumindest ähnlich sind. Wie in Fig. 5 erkennbar ist, wird das erste Fluid F1 zuerst auf dem Zielobjekt abgeschieden. Das zweite Fluid F2 wird anschließend auf das erste Fluid F1 aufgetragen. Schließlich kann ein drittes Fluid F3 nach Beendigung des Fluidstrahls des Fluids F2 auf den Schichtverbund des ersten Fluids F1 und des zweiten Fluids F2 abgeschieden werden. Das dritte Fluid F3 kann beispielsweise, wie dies in dem Diagramm gemäß Fig. 5 erkennbar ist, mit einem ähnlichen oder identischen Volumenstrom über die Düse versprüht werden wie das zweite Fluid F2. Im Unterschied zu dem zweiten Fluid F2 kann jedoch die Sprühdauer für das dritte Fluid F3 verlängert sein. Durch die Einstellung der unterschiedlichen Volumenströme der Fluide F1, F2, F3 kann den unterschiedlichen Viskositäten der Fluide F1, F2, F3 Rechnung getragen werden. Auf diese Weise wird erreicht, dass die Fluide F1, F2, F3 trotz ihrer unterschiedlichen Viskosität mit derselben Strömungsgeschwindigkeit aus der Düse austreten. Im Übrigen kann vorgesehen sein, dass die Fluide F1, F2, F3 jeweils aus unterschiedlichen Kanalsystemen 5 der Düse austreten, so dass eine Vermischung der Fluide F1, F2, F3 erst auf dem Zielobjekt erfolgt. Der Schichtaufbau gemäß Fig. 5 ist daher im Wesentlichen beispielhaft und allenfalls für einen Augenblick erkennbar. In der Praxis erfolgt auf dem Zielobjekt unmittelbar eine Vermischung der einzelnen Fluide.

Die zuvor beschriebene Düse dient zum Vermischen und Versprühen von Fluiden. Dabei können Fluide mit gleichen oder unterschiedlichen Volumenströmen zugeführt werden und/oder gleiche oder unterschiedliche Viskosität aufweisen. Der Begriff Fluid umfasst hierbei sowohl flüssige, als auch gasförmige Substanzen, sowie deren Gemische. Insbesondere kann mit der erfindungsgemäßen Düse ein Zweikomponentenkleber vermischt und versprüht werden, wobei durch die außenmischende Funktion der Düse ein Verkleben der Düsenkanäle vermieden wird. Ein derartiger Zweikomponentenkleber weist üblicherweise ein Bindemittel und ein Härtemittel bzw. Vernetzungsmittel auf. Dabei wird das Härtemittel bzw. Vernetzungsmittel vorzugsweise als erstes Fluid über den ersten Zufuhrkanal 11, die erste Kammer 12 und den ersten Austrittkanal 10 versprüht. Als Härtemittel bzw. Vernetzungsmittel kann beispielsweise Thrombin eingesetzt werden. Das Bindemittel wird vorzugsweise als zweites Fluid über den zweiten Zufuhrkanal 21, die zweite Kammer 22 und den zweiten Austrittskanal 20 versprüht. Ein bevorzugtes Bindemittel ist beispielsweise Fibrinogen. Das Bindemittel und das Härtemittel bzw. Vernetzungsmittel gelangen erst im Überlappungsbereich 34 miteinander in Kontakt, so dass die Vernetzungsreaktion bzw. Aushärtung außerhalb der Düse stattfindet. Innerhalb der Düse werden das erste Fluid und das zweite Fluid, insbesondere das Bindemittel und das Härtemittel bzw. Vernetzungsmittel, vollständig getrennt voneinander geführt.

Zusätzlich kann als drittes Fluid eine Substanz zugeführt werden, die beispielsweise biologisches Material, insbesondere Zellen, aufweist. Um das biologische Material zu schonen, ist vorgesehen, dass das dritte Fluid weitgehend umlenkungsfrei, also unter Einfluss möglichst geringer Scherkräfte, zugemischt wird. Dies kann einerseits durch die koaxiale Anordnung des dritten Zufuhrkanals 31 zur Mischkammer 22b gemäß Fig. 1 und andererseits durch einen separaten Austrittskanal 30 erfolgen, in dem der dritte Zufuhrkanal 31 koaxial mündet (Fig. 2). Die Zuführung des dritten Fluids seitlich in den zweiten Austrittskanal 20 gemäß Fig. 3 schont ebenfalls biologisches Gewebe, das mit dem dritten Fluid zugemischt wird.

Für alle Ausführungsbeispiele gilt, dass die erfindungsgemäße Düse vorzugsweise volumenstromangepasste Querschnitte der Eintrittsöffnungen 13, 23 aufweist, um die unterschiedliche Viskosität und/oder die unterschiedlichen Volumenströme der einzelnen Fluide auszugleichen. Ferner ist es möglich, anstelle einzelner, unterschiedlich großer Eintrittsöffnungen, eine unterschiedliche Anzahl von Eintrittsöffnungen 13, 23 vorzusehen. So kann beispielsweise die erste Kammer 12 eine größere Anzahl von Eintrittsöffnungen 13 als die zweite Kammer 22 aufweisen, oder umgekehrt. Ferner können die Querschnitte der Austrittskanäle 10, 20 in Abhängigkeit des Volumenstromverhältnisses der einzelnen Fluide gewählt sein, um im Wesentlichen gleiche mittlere Strömungsgeschwindigkeiten der aus der Düse austretenden Fluide einzustellen. Durch eine Neigung der Mittelachsen der Austrittskanäle 10, 20 kann ferner der Überlappungsbereich 34 vergrößert werden, um die Vermischung der einzelnen Fluide zu verbessern. Vorzugsweise besteht zwischen den Mittelachsen der Austrittskanäle 10, 20 ein Winkel, der größer als 0° und kleiner als 180° ist.

Im Rahmen der Anmeldung wird ferner ein Verfahren zum Mischen von wenigstens zwei Fluiden mittels einer außenmischenden Düse für medizinische Zwecke offenbart, die wenigstens zwei Austrittskanäle 10, 20 und wenigstens zwei Eintrittsöffnungen 13, 23 mit unterschiedlichen oder gleichen Querschnitten aufweist, wobei zwei Fluide mit unterschiedlichen Volumenströmen und/oder unterschiedlicher Viskosität versprüht werden, und wobei das Verhältnis der Querschnitte der Eintrittskanäle 13, 23 und/oder der Austrittskanäle 10, 20 dem Verhältnis der Volumenströme entspricht, so dass die Fluide mit im Wesentlichen gleichen mittleren Strömungsgeschwindigkeiten durch die Austrittskanäle 10, 20 und/oder die Eintrittsöffnungen 13, 23 fließen. Außerdem werden eine außenmischende Düse, ein medizinisches Instrument und ein medizinisches Gerät zum Versprühen von Substanzen, insbesondere biologischem Material beschrieben.

### Bezugszeichenliste

- 5: Kanalsystem
- 10: erster Austrittskanal
- 11: erster Zufuhrkanal
- 11a: Endabschnitt des ersten Zufuhrkanals 11
- 12: erste Kammer
- 12a: erste Drallkammer
- 13: erste Eintrittsöffnung
- 14: erster Fluidkegel
- 15: Engstelle
- 20: zweiter Austrittskanal
- 21: zweiter Zufuhrkanal
- 21a: Endabschnitt des zweiten Zufuhrkanals 21
- 22: zweite Kammer
- 22a: zweite Drallkammer
- 22b: Mischkammer
- 23: zweite Eintrittsöffnung
- 24: zweiter Fluidkegel
- 30: dritter Austrittskanal
- 31, 41: dritter Zufuhrkanal
- 33: Punktstrahl
- 34: Überlappungsbereich
- 40: Endfläche
- 41a: Endabschnitt des dritten Zufuhrkanals 41

## Patentansprüche

1. Verfahren zum Mischen von wenigstens zwei Fluiden mittels einer außenmischenden Düse für medizinische Zwecke, die wenigstens zwei Austrittskanäle (10, 20) und wenigstens zwei Eintrittsöffnungen (13, 23) mit unterschiedlichen oder gleichen Querschnitten aufweist, wobei
- ein erstes Fluid über einen ersten Zufuhrkanal (11) seitlich, insbesondere tangential, in eine erste Kammer (12) geleitet wird, die mit einem ersten Austrittskanal (10) fluidverbunden ist,
- ein zweites Fluid über einen zweiten Zufuhrkanal (21) seitlich, insbesondere tangential, in eine zweite Kammer (22) geleitet wird, die mit einem zweiten Austrittskanal (20) fluidverbunden ist,
- das erste Fluid über den ersten Austrittskanal (10) und das zweite Fluid über den zweiten Austrittskanal (20) ausströmen derart, dass sich überlappende Fluidkegel bilden,
wobei das erste Fluid und das zweite Fluid unterschiedliche Volumenströme und/oder unterschiedliche Viskosität aufweisen und das Verhältnis der Querschnitte der Eintrittsöffnungen (13, 23) und/oder der Austrittskanäle (10, 20) dem Verhältnis der Volumenströme entspricht, so dass das erste Fluid und das zweite Fluid mit im Wesentlichen gleichen mittleren Strömungsgeschwindigkeiten durch die Austrittskanäle (10, 20) und/oder die Eintrittsöffnungen (13,23) fließen.

2. Verfahren nach Anspruch 1,
**dadurch gekennzeichnet, dass**
die Zufuhr des ersten Fluids zur ersten Kammer (12) und/oder die Zufuhr des zweiten Fluids zur zweiten Kammer (22) gesteuert wird.

3. Verfahren nach Anspruch 1 oder 2,
**dadurch gekennzeichnet, dass**
ein drittes Fluid durch einen dritten Zufuhrkanal (31) in die zweite Kammer (22) geleitet und mit dem zweiten Fluid vermischt wird.

4. Verfahren nach Anspruch 3,
**dadurch gekennzeichnet, dass**
das dritte Fluid koaxial in die zweite Kammer (22) einströmt.

5. Verfahren nach Anspruch 1 oder 2,
**dadurch gekennzeichnet, dass**
ein drittes Fluid über einen dritten Austrittskanal (30) in die überlappenden Fluidkegel des ersten Fluids und des zweiten Fluids geleitet wird.

6. Verfahren nach Anspruch 1 oder 2,
**dadurch gekennzeichnet, dass**
ein drittes Fluid direkt, insbesondere seitlich, in den zweiten Austrittskanal (20) eingeleitet und dort mit dem zweiten Fluid vermischt wird.

7. Außenmischende Düse zur Zufuhr von Substanzen, insbesondere biologischem Material, mit einem ersten Austrittskanal (10) und einem zweiten Austrittskanal (20), die beabstandet zueinander angeordnet sind derart, dass sich aus den ersten und zweiten Austrittskanälen (10, 20) austretende Fluidkegel zur Vermischung von Fluiden zumindest teilweise überlappen, wobei der erste Austrittskanal (10) mit einer ersten Kammer (12) und der zweite Austrittskanal (20) mit einer zweiten Kammer (22) fluidverbunden sind und ein erster Zufuhrkanal (11) seitlich, insbesondere tangential, in die erste Kammer (12) und ein zweiter Zufuhrkanal (21) seitlich, insbesondere tangential, in die zweite Kammer (22) mündet,
**dadurch gekennzeichnet, dass**
wenigstens ein dritter Zufuhrkanal (31) vorgesehen ist, der koaxial in die zweite Kammer (22) oder direkt in den zweiten Austrittskanal (20) oder einen dritten Austrittskanal (30) mündet.

8. Düse nach Anspruch 7,
**dadurch gekennzeichnet, dass**
der dritte Zufuhrkanal (31) seitlich in den zweiten Austrittskanal (20) mündet oder koaxial zum zweiten Austrittskanal (20) angeordnet ist.

9. Düse nach Anspruch 7 oder 8,
**dadurch gekennzeichnet, dass**
der dritte Austrittskanal (31) zwischen dem ersten und dem zweiten Austrittskanal (10, 20) angeordnet ist derart, dass ein aus dem dritten Austrittskanal (30) austretendes Fluid in die überlappenden Fluidkegel geleitet wird.

10. Düse nach einem der vorhergehenden Ansprüche,
**dadurch gekennzeichnet, dass**
die erste Kammer (12) und die zweite Kammer (22) jeweils mit dem ersten oder zweiten Zufuhrkanal (11, 21) über eine Eintrittsöffnung (13, 23) verbunden sind, wobei die Eintrittsöffnung (13) der ersten Kammer (12) und die Eintrittsöffnung (23) der zweiten Kammer (22) unterschiedliche Querschnittsflächen aufweisen.

11. Düse nach einem der vorhergehenden Ansprüche,
**dadurch gekennzeichnet, dass**
wenigstens der erste Austrittskanal (10) und der zweite Austrittskanal (20), insbesondere alle Austrittskanäle (10, 20, 30), parallel oder winklig zueinander ausgerichtete Längsachsen aufweisen.

12. Düse nach einem der vorhergehenden Ansprüche,
**dadurch gekennzeichnet, dass**
wenigstens der zweite Austrittskanal (20) eine Engstelle (15) aufweist, insbesondere wobei der dritte Zufuhrkanal (31) im Bereich der Engstelle (15) in den zweiten Austrittskanal (20) mündet.

13. Medizinisches Instrument mit einer außenmischenden Düse nach einem der Ansprüche 7 bis 12, wobei das Instrument an ein medizinisches Gerät mit einer Steuerung oder Regelung zur Einstellung der Fluidzufuhr anschließbar ist.

14. Medizinisches Gerät mit einer außenmischenden Düse nach einem der Ansprüche 7 bis 12 und/oder einem Instrument nach Anspruch 13,
**gekennzeichnet durch**
eine Steuerung oder Regelung, die zur Einstellung der Fluidzufuhr angepasst ist derart, dass bei unterschiedlichen Volumenströmen und/oder unterschiedlicher Viskosität der Fluide diese mit im Wesentlichen gleichen Strömungsgeschwindigkeiten durch die Austrittskanäle (10,20) und/oder die Eintrittsöffnungen (13, 23) fließen.

15. Medizinisches Gerät mit einer außenmischenden Düse nach einem der Ansprüche 7 bis 12 und/oder einem Instrument nach Anspruch 13, insbesondere ein Gerät nach Anspruch 14,
**gekennzeichnet durch**
eine Steuerung oder Regelung, die zur Einstellung der Fluidzufuhr angepasst ist derart, dass die unterschiedlichen Fluide unabhängig voneinander, in einer beliebigen Reihenfolge zugeführt werden können.

## Claims

1. Method for mixing at least two fluids by means of an externally mixing nozzle for medical purposes, which has at least two outlet channels (10, 20) and at least two inlet openings (13, 23) with different or identical cross sections, wherein
- a first fluid is conveyed laterally, in particular tangentially, via a first supply channel (11) into a first chamber (12) which is fluidically connected to a first outlet channel (10),
- a second fluid is conveyed laterally, in particular tangentially, via a second supply channel (21) into a second chamber (22) which is fluidically connected to a second outlet channel (20),
- via the first outlet channel (10), the first fluid, and via the second outlet channel (20), the second fluid flow out in such a manner that overlapping fluid cones form,
wherein the first fluid and the second fluid have different volumetric flow rates and/or different viscosity and the ratio of the cross sections of the inlet openings (13, 23) and/or of the outlet channels (10, 20) corresponds to the ratio of the volumetric flow rates, so that the first fluid and the second fluid flow through the outlet channels (10, 20) and/or the inlet openings (13, 23) at substantially equal average flow speeds.

2. Method according to Claim 1,
**characterized in that**
the supply of the first fluid to the first chamber (12) and/or the supply of the second fluid to the second chamber (22) is controlled.

3. Method according to Claim 1 or 2,
**characterized in that**
a third fluid is conveyed through a third supply channel (31) into the second chamber (22) and is mixed with the second fluid.

4. Method according to Claim 3,
**characterized in that**
the third fluid flows coaxially into the second chamber (22).

5. Method according to Claim 1 or 2,
**characterized in that**
a third fluid is conveyed via a third outlet channel (30) into the overlapping fluid cones of the first fluid and of the second fluid.

6. Method according to Claim 1 or 2,
**characterized in that**
a third fluid is introduced directly, in particular laterally, into the second outlet channel (20), where it is mixed with the second fluid.

7. Externally mixing nozzle for supplying substances, in particular biological material, having a first outlet channel (10) and a second outlet channel (20) which are arranged at a distance from one another in such a manner that fluid cones exiting from the first and second outlet channels (10, 20) for mixing fluids at least partially overlap, wherein the first outlet channel (10) is fluidically connected to a first chamber (12) and the second outlet channel (20) is fluidically connected to a second chamber (22) and a first supply channel (11) opens laterally, in particular tangentially, into the first chamber (12) and a second supply channel (21) opens laterally, in particular tangentially, into the second chamber (22),
**characterized in that**
there is provided at least one third supply channel (31) which opens coaxially into the second chamber (22) or directly into the second outlet channel (20) or a third outlet channel (30).

8. Nozzle according to Claim 7,
**characterized in that**
the third supply channel (31) opens laterally into the second outlet channel (20) or is arranged coaxially with respect to the second outlet channel (20).

9. Nozzle according to Claim 7 or 8,
**characterized in that**
the third outlet channel (31) is arranged between the first and the second outlet channel (10, 20) in such a manner that a fluid which exits from the third outlet channel (30) is conveyed into the overlapping fluid cones.

10. Nozzle according to one of the preceding claims,
**characterized in that**
the first chamber (12) and the second chamber (22) are in each case connected via an inlet opening (13, 23) to the first or second supply channel (11, 21), wherein the inlet opening (13) of the first chamber (12) and the inlet opening (23) of the second chamber (22) have different cross-sectional areas.

11. Nozzle according to one of the preceding claims,
**characterized in that**
at least the first outlet channel (10) and the second outlet channel (20), in particular all outlet channels (10, 20, 30), have longitudinal axes which are aligned parallel or at an angle with respect to one another.

12. Nozzle according to one of the preceding claims,
**characterized in that**
at least the second outlet channel (20) has a constriction (15), in particular wherein, in the region of the constriction (15), the third supply channel (31) opens into the second outlet channel (20).

13. Medical instrument having an externally mixing nozzle according to one of Claims 7 to 12, wherein the instrument is connectable to a medical appliance having a controller or regulator for setting the fluid supply.

14. Medical appliance having an externally mixing nozzle according to one of Claims 7 to 12 and/or having an instrument according to Claim 13,
**characterized by**
a controller or regulator which, for the purpose of setting the fluid supply, is adapted in such a manner that, in the case of different volumetric flow rates and/or different viscosity of the fluids, said fluids flow through the outlet channels (10, 20) and/or the inlet openings (13, 23) at substantially equal flow speeds.

15. Medical appliance having an externally mixing nozzle according to one of Claims 7 to 12 and/or having an instrument according to Claim 13, in particular an appliance according to Claim 14,
**characterized by**
a controller or regulator which, for the purpose of setting the fluid supply, is adapted in such a manner that the different fluids can be supplied, independently of one another, in any desired sequence.

## Revendications

1. Procédé pour mélanger au moins deux fluides au moyen d'une buse à mélange extérieur pour usages médicaux, qui présente au moins deux canaux de sortie (10, 20) et au moins deux ouvertures d'entrée (13, 23) ayant des sections transversales différentes ou identiques,
- un premier fluide étant guidé par le biais d'un premier canal d'alimentation (11) latéralement, en particulier tangentiellement, dans une première chambre (12) qui est en liaison fluidique avec un premier canal de sortie (10),
- un deuxième fluide étant guidé par le biais d'un deuxième canal d'alimentation (21) latéralement, en particulier tangentiellement, dans une deuxième chambre (22) qui est en liaison fluidique avec un deuxième canal de sortie (20),
- le premier fluide s'écoulant par le biais du premier canal de sortie (10) et le deuxième fluide s'écoulant par le biais du deuxième canal de sortie (20) de telle sorte qu'il se forme des cônes de fluide se chevauchant,
le premier fluide et le deuxième fluide présentant des débits volumiques différents et/ou une viscosité différente et le rapport des sections transversales des ouvertures d'entrée (13, 23) et/ou des canaux de sortie (10, 20) correspondant au rapport des débits volumiques, de telle sorte que le premier fluide et le deuxième fluide s'écoulent avec des vitesses d'écoulement moyennes essentiellement identiques à travers les canaux de sortie (10, 20) et/ou les ouvertures d'entrée (13, 23) .

2. Procédé selon la revendication 1,
**caractérisé en ce que** l'alimentation du premier fluide à la première chambre (12) et/ou l'alimentation du deuxième fluide à la deuxième chambre (22) est commandée.

3. Procédé selon la revendication 1 ou 2,
**caractérisé en ce qu'**
un troisième fluide est guidé à travers un troisième canal d'alimentation (31) dans la deuxième chambre (22) et est mélangé au deuxième fluide.

4. Procédé selon la revendication 3,
**caractérisé en ce que**
le troisième fluide afflue coaxialement dans la deuxième chambre (22).

5. Procédé selon la revendication 1 ou 2,
**caractérisé en ce qu'**
un troisième fluide est guidé par le biais d'un troisième canal de sortie (30) dans les cônes de fluide se chevauchant du premier fluide et du deuxième fluide.

6. Procédé selon la revendication 1 ou 2,
**caractérisé en ce qu'**
un troisième fluide est introduit directement, en particulier latéralement, dans le deuxième canal de sortie (20) et est mélangé au deuxième fluide.

7. Buse à mélange extérieur pour l'alimentation de substances, en particulier de matière biologique, comprenant un premier canal de sortie (10) et un deuxième canal de sortie (20) qui sont disposés à distance l'un de l'autre de telle sorte que des cônes de fluide sortant du premier et du deuxième canal de sortie (10, 20) pour le mélange de fluides se chevauchent au moins en partie, le premier canal de sortie (10) étant en liaison fluidique avec une première chambre (12) et le deuxième canal de sortie (20) étant en liaison fluidique avec une deuxième chambre (22) et un premier canal d'alimentation (11) débouchant latéralement, en particulier tangentiellement, dans la première chambre (12) et un deuxième canal d'alimentation (21) débouchant latéralement, en particulier tangentiellement, dans la deuxième chambre (22),
**caractérisée en ce qu'**
au moins un troisième canal d'alimentation (31) est prévu, lequel débouche coaxialement dans la deuxième chambre (22) ou directement dans le deuxième canal de sortie (20) ou dans un troisième canal de sortie (30).

8. Buse selon la revendication 7,
**caractérisée en ce que**
le troisième canal d'alimentation (31) débouche latéralement dans le deuxième canal de sortie (20) ou est disposé coaxialement par rapport au deuxième canal de sortie (20).

9. Buse selon la revendication 7 ou 8,
**caractérisée en ce que**
le troisième canal de sortie (31) est disposé entre le premier et le deuxième canal de sortie (10, 20) de telle sorte qu'un fluide sortant du troisième canal de sortie (30) soit guidé dans les cônes de fluide se chevauchant.

10. Buse selon l'une quelconque des revendications précédentes,
**caractérisée en ce que**
la première chambre (12) et la deuxième chambre (22) sont à chaque fois connectées au premier ou au deuxième canal d'alimentation (11, 21) par le biais d'une ouverture d'entrée (13, 23), l'ouverture d'entrée (13) de la première chambre (12) et l'ouverture d'entrée (23) de la deuxième chambre (22) présentant des surfaces en section transversale différentes.

11. Buse selon l'une quelconque des revendications précédentes,
**caractérisée en ce qu'**
au moins le premier canal de sortie (10) et le deuxième canal de sortie (20), en particulier tous les canaux de sortie (10, 20, 30) présentent des axes longitudinaux orientés parallèlement ou de manière angulaire les uns par rapport aux autres.

12. Buse selon l'une quelconque des revendications précédentes,
**caractérisée en ce qu'**
au moins le deuxième canal de sortie (20) présente un point d'étranglement (15), en particulier le troisième canal d'alimentation (31) débouchant dans la région du point d'étranglement (15) dans le deuxième canal de sortie (20).

13. Instrument médical comprenant une buse à mélange extérieur selon l'une quelconque des revendications 7 à 12, dans lequel l'instrument peut être raccordé à un appareil médical comprenant une commande ou une régulation pour l'ajustement de l'alimentation en fluide.

14. Appareil médical comprenant une buse à mélange extérieur selon l'une quelconque des revendications 7 à 12 et/ou un instrument selon la revendication 13,
**caractérisé par**
une commande ou une régulation qui est adaptée pour l'ajustement de l'alimentation en fluide de telle sorte qu'en cas de débits volumiques différents et/ou d'une viscosité différente des fluides, ceux-ci s'écoulent avec des vitesses d'écoulement essentiellement identiques à travers les canaux de sortie (10, 20) et/ou les ouvertures d'entrée (13, 23).

15. Appareil médical comprenant une buse à mélange extérieur selon l'une quelconque des revendications 7 à 12 et/ou instrument selon la revendication 13, en particulier un appareil selon la revendication 14,
**caractérisé par**
une commande ou une régulation qui est adaptée pour l'ajustement de l'alimentation en fluide de telle sorte que les différents fluides puissent être alimentés indépendamment les uns des autres dans une séquence quelconque.
